# EUROPEAN PATENT APPLICATION

(11) **EP 3 702 837 A1**
(43) Date of publication of application: **02.09.2020**
(21) Application number: 18871767.2
(22) Date of filing: 23.10.2018
(51) Int. Cl.: G03F 7/00, G02B 27/28, G02B 21/36

(54) **PHOTOLITHOGRAPHY METHOD, PHOTOLITHOGRAPHY PRODUCT AND PHOTOLITHOGRAPHY MATERIAL**

(30) Priority: 23.10.2017 CN 201710995525
(71) Applicant: Shanghai Bixiufu Enterprise Management Co., Ltd., Shanghai 201112 (CN)
(72) Inventor: WANG, Lijiang, Shanghai 201112 (CN); WANG, Wei, Shanghai 201112 (CN); ZHU, Song, Shanghai 201112 (CN)
(74) Representative: de Rooij, Mathieu Julien
(86) International application number: PCT/CN2018/111325
(87) International publication number: WO 2019/080820

(57) **Abstract**

This invention relates to the field of lithography and particularly to a lithographic method, a lithographic product and a lithographic material. The invention provides a lithographic method including the steps of: 1) providing first light and second light to the lithographic material, wherein at least part of molecules for generating effector molecules controllable by a molecular switch in a turned-on state generate effector molecules, thereby changing physical and/or chemical properties of the lithographic material in an area where the molecular switch is turned on; and 2) removing either the lithographic material that has changed in physical or chemical properties or the lithographic material that has not changed. The novel lithographic method provided by the invention can effectively break through the diffraction limit of light, thereby further improving lithography precision.

## Description

### TECHNICAL FIELD

The invention relates to the field of lithography and in particular to a lithographic method and a lithographic material.

### BACKGROUND ART

Lithography is an important technical link in current industrial precision machining. In particular, lithography has wide application in the field of micro-nano machining, such as integrated circuit chips, MEMS devices, optical integration technology, and precision optics. At present, the mainstream high-precision lithography manufacturing processes mainly include optical projection micro-lithography, electron light beam direct writing, ion light beam machining, laser interference lithography, and the like.

According to the Rayleigh resolution equation R=kl λ/NA, it is known that an increase in resolution can be achieved by increasing the numerical aperture NA of a lithography objective lens and shortening the exposure wavelength λ. At present, immersion lithography is adopted as a method for increasing the numerical aperture for a lithography machine, an ArF light source with a wavelength of 193nm is adopted as the most popular and mature means of exposure to short-wavelength light, and the minimum resolution cannot break through 45nm even if the method of improving NA by immersion lithography is adopted.

Extreme ultraviolet (EUV), multi-light-beam maskless and nanoimprint are currently considered to be the most promising candidates for next generation lithography (NGL). The most obvious feature of EUV technology is that the exposure wavelength can be shortened to 13.5nm, which greatly improves the resolution. However, under such a short-wavelength light source, almost all substances have strong absorptivity, and hence a conventional transmissive optical system cannot be used. A reflective optical system must be used instead, but it is difficult to design a reflective optical system having a large NA. As a result, the resolution cannot be improved. Moreover, a reflective optical system is difficult to manufacture because EUV masks adopt a reflective type (typically transmissive type). In addition, difficulties exist concerning storage, shipment and operation of the masks.

In maskless lithography, an electron light beam (EB) has the characteristics of a short wavelength, high resolution, long focal depth, easy control and flexibility in modification. Therefore, it is widely used in optical and non-optical mask manufacture. In the development of system-on-a-chip (SOC), electron light beam direct writing (EBDW) is more flexible than other methods. EBDW can directly accept graphics data imaging without complex mask manufacture, and therefore it has broad application prospects. However, low productivity of EBDW limits its use.

The existing exposure-based lithography technology features an improvement of the resolution on the basis of the Rayleigh equation by adopting conventional optical principles, mainly by adopting the immersion method to improve the numerical aperture and adopting a shorter-wavelength light source, especially the latter which plays a decisive role in improving the resolution. However, existing exposure-based lithography technology is complex and difficult to manufacture an excimer light source, the electron light beam, and even the extreme ultraviolet light source which are extremely costly. Therefore a direct super-resolution exposure lithographic machine is very expensive, and the resolution is still limited by the diffraction limit of the light source.

### SUMMARY OF THE INVENTION

In view of the above-mentioned disadvantages of the prior art, it is an object of the present invention to provide a lithographic method and lithographic material for solving the problems of the prior art.

In order to achieve the above object and other related objects, a first aspect of the present invention provides a lithographic method, comprising the steps of:
1) providing first light and second light to a lithographic material, wherein the first light and the second light partially overlap, the lithographic material contains molecules for generating effector molecules controllable by a molecular switch, the first light is used for enabling the molecules for generating effector molecules controllable by the molecular switch to be in a turned-off state, and the second light is used for enabling the molecules for generating effector molecules controllable by the molecular switch to be in a turned-on state; the molecules for generating effector molecules controllable by the molecular switch in an area where the first light and the second light overlap are in the turned-off state; at least part of the molecules for generating effector molecules controllable by the molecular switch in the turned-on state generate effector molecules, thereby changing physical and/or chemical properties of the lithographic material in the area where the molecular switch is turned on; and
2) removing either the lithographic material that has changed in physical or chemical properties or the lithographic material that has not changed.

In some embodiments of the present application, the first light is single hollow light or multiple hollow light.

In some embodiments of the present application, the second light at least partially covers a non-illuminated area surrounded by an area illuminated by the first light.

In some embodiments of the present application, the second light is solid light.

In some embodiments of the present application, at least one of the first light and the second light is array light.

In some embodiments of the present application, an area illuminated by the second light does not exceed an outer edge of the area illuminated by the first light.

In some embodiments of the present application, at least one of the first light and the second light comprises multiple light beams.

In some embodiments of the present application, the effector molecules generated by the molecules for generating effector molecules controllable by the molecular switch are selected from the group consisting of molecules for removing protecting groups from the lithographic material and molecules for activating polymerization control of the lithographic material.

In some embodiments of the present application, the molecules for removing protecting groups from the lithographic material are selected from acidic molecules, basic molecules, and singlet oxygen.

In some embodiments of the present application, the molecules for activating polymerization control of the lithographic material are selected from polymerization initiation molecules.

In some embodiments of the present application, the molecule for removing effector molecules controllable by the molecular switch comprises a molecular switch group and an effector molecule generating group in the molecular structure thereof.

In some embodiments of the present application, in the molecule for removing effector molecules controllable by the molecular switch, the molecular switch group is linked to the effector molecule generating group through a chemical bond.

In some embodiments of the present application, in step 1), the manner in which the molecules for generating effector molecules controllable by the molecular switch in the turned-on state generate the effector molecules is selected from the group consisting of changing illumination conditions to which the molecules for generating effector molecules controllable by the molecular switch in the turned-on state are subjected.

In some embodiments of the present application, a change in physical or chemical properties of the lithographic material refers to a change in solubility in a developing solution.

In some embodiments of the present application, a method of changing the physical or chemical properties of the lithographic material is selected from the group consisting of removing protecting groups from the lithographic material or polymerizing polymer monomers in the lithographic material.

A second aspect of the invention provides a lithographic material comprising molecules for generating effector molecules controllable by a molecular switch and a compound sensitive to effector molecules.

In some embodiments of the present application, the effector molecules are selected from the group consisting of molecules for removing protecting groups from the lithographic material and molecules for activating polymerization control of the lithographic material. The molecules for removing protecting groups from the lithographic material are selected from acidic molecules, basic molecules, and singlet oxygen.

In some embodiments of the present application, the molecules for activating polymerization control of the lithographic material are selected from polymerization initiation molecules.

In some embodiments of the present application, the compound sensitive to the effector molecules is selected from the group consisting of a polymer sensitive to the effector molecules, a polymerized monomer, and an oligomer sensitive to the effector molecules.

In some embodiments of the present application, the molecule for generating effector molecules controllable by the molecular switch comprises a molecular switch group and an effector molecule generating group.

In some embodiments of the present application, in the molecule for generating effector molecules controllable by the molecular switch, the molecular switch group is linked to the effector molecule generating group through a chemical bond.

A third aspect of the invention provides a compound comprising a molecular switch group and an effector molecule generating group.

In some embodiments of the present application, the effector molecules are selected from the group consisting of molecules for removing protecting groups from the lithographic material and molecules for activating polymerization control of the lithographic material.

In some embodiments of the present application, the molecules for removing protecting groups from the lithographic material are selected from acidic molecules, basic molecules, and singlet oxygen.

In some embodiments of the present application, the molecules for activating polymerization control of the lithographic material are selected from polymerization initiation molecules.

In some embodiments of the present application, in the molecule for generating effector molecules controllable by the molecular switch, the molecular switch group is linked to the effector molecule generating group through a chemical bond.

### BRIEF DESCRIPTION OF THE DRAWINGS

FIG. 1 is a process diagram of basic principles for implementing super-resolution using a dual-beam technology;
FIG. 2 is diagrams of patterns of polymerization deprotection controllable by a molecular switch;
FIGS. 3A and 3B are diagrams of patterns of polymerization of monomers controllable by the molecular switch;
FIGS. 4A and 4B show forms of reactions of typical molecular switches;
FIG. 5 shows a group-removing reaction pattern of a molecular switch of a pericyclic reaction system;
FIG. 6 is diagrams of a pattern of positive dual-beam lithography by means of polymerization deprotection controllable by the molecular switch;
FIG. 7 is diagrams of a pattern of negative dual-beam lithography by means of monomer polymerization controllable by the molecular switch;
FIGS. 8A and 8B are diagrams of a process of positive dual-beam lithography by means of polymerization deprotection controllable by the molecular switch;
FIGS. 9A and 9B are diagrams of a process of negative dual-beam lithography by means of monomer polymerization controllable by the molecular switch;
FIGS. 10A and 10B are diagrams of patterns of dual-beam shaping;
FIGS. 11A and 11B show two types of solutions of a parallelized dual-beam lithographic system; and
FIG. 12 shows a scanning mode of a dual-beam array, including rectangular coordinate system scanning and polar coordinate system scanning.

### DETAILED DESCRIPTION OF THE INVENTION

Through a great deal of exploratory research, the inventor found a new lithographic method and further provides a lithographic material corresponding to the lithographic method. The lithographic method and the lithographic material can effectively break through the diffraction limit of light so that the lithography precision is further improved.

### Lithographic method

An aspect of the present application provides a lithographic method, including providing first light and second light to the lithographic material, wherein the first light and the second light can partially overlap, the lithographic material contains molecules for generating effector molecules controllable by a molecular switch, and at least part of the molecules for generating effector molecules controllable by the molecular switch in a turned-on state generate effector molecules, thereby changing physical and/or chemical properties of the lithographic material in an area where the molecular switch is turned on.

In the lithographic method, the second light can be used for enabling the molecules for generating effector molecules controllable by the molecular switch to be in the turned-on state, and a person skilled in the art would be able to determine conditions for enabling the molecules for generating effector molecules controllable by the molecular switch to be in the turned-on state according to parameters such as the type of the molecules for generating effector molecules controllable by the molecular switch, the content thereof in the lithographic material, and the like. For example, the second light can be ultraviolet light, visible light or infrared light, and the like. As another example, the second light can have a wavelength of not greater than 5nm, 5-10nm, 10-20nm, 20-40nm, 40-60nm, 60-80nm, 80-100nm, 100-150nm, 150-200nm, 200-250nm, 250-300nm, 300-350nm, 350-400nm, 400-450nm, 450-500nm, 500-550nm, 550-600nm, 600-650nm, 650-700nm, 700-750nm, 750-800nm, 800-850nm, 850-900nm, 900-950nm, 950-1000nm, 1000-1200nm, 1200-1400nm, 1400-1600nm, 1600-1800nm, 1800-2000nm, 2000-2500nm, 2500-3000nm, 3000-3500nm, 3500-4000nm, 4000-4500nm, 4500-5000nm, 5000-6000nm, 6000-7000nm, 7000-8000nm, 8000-9000nm, 8000-9000nm, 8000-9000nm, 8000-9000nm, 8000-9000nm, 8000-9000nm, 9000-10000nm, 10000-12000nm, 12000-14000nm, 14000-16000nm, 16000-18000nm, 18000-20000nm, or not less than 20000nm.

In the lithographic method, the first light can be used for enabling the molecules for generating effector molecules controllable by the molecular switch to be in a turned-off state, the area where the first light and the second light overlap can normally enable the molecules for generating effector molecules controllable by the molecular switch to be in the turned-off state, and enabling the molecules for generating effector molecules controllable by the molecular switch to be in the turned-off state can be, for example, switching the molecules for generating effector molecules controllable by the molecular switch in the turned-on state into the turned-off state and/or keeping the molecules for generating effector molecules controllable by the molecular switch in the turned-off state still in the turned-off state. A person skilled in the art would be able to determine conditions for enabling the molecules for generating effector molecules controllable by the molecular switch to be in the turned-off state according to parameters such as the type of the molecules for generating effector molecules controllable by the molecular switch, the content thereof in the lithographic material, and the like. For example, the first light can be ultraviolet light, visible light or infrared light, and the like. As another example, the first light can have a wavelength of not greater than 5nm, 5-10nm, 10-20nm, 20-40nm, 40-60nm, 60-80nm, 80-100nm, 100-150nm, 150-200nm, 200-250nm, 250-300nm, 300-350nm, 350-400nm, 400-450nm, 450-500nm, 500-550nm, 550-600nm, 600-650nm, 650-700nm, 700-750nm, 750-800nm, 800-850nm, 850-900nm, 900-950nm, 950-1000nm, 1000-1200nm, 1200-1400nm, 1400-1600nm, 1600-1800nm, 1800-2000nm, 2000-2500nm, 2500-3000nm, 3000-3500nm, 3500-4000nm, 4000-4500nm, 4500-5000nm, 5000-6000nm, 6000-7000nm, 7000-8000nm, 8000-9000nm, 8000-9000nm, 8000-9000nm, 8000-9000nm, 8000-9000nm, 8000-9000nm, 9000-10000nm, 10000-12000nm, 12000-14000nm, 14000-16000nm, 16000-18000nm, 18000-20000nm, or not less than 20000nm.

In the lithographic method, generally speaking, for the same effector molecules controllable by the molecular switch, the conditions for enabling the effector molecules controllable by the molecular switch to be in the turned-on state and the turned-off state are different, and a person skilled in the art would be able to determine the conditions for enabling the molecules for generating effector molecules controllable by the molecular switch to be in different states according to the type of the effector molecules controllable by the molecular switch, such as the conditions for enabling the molecules for generating effector molecules controllable by the molecular switch to be in the turned-on state, for enabling the molecules for generating effector molecules controllable by the molecular switch to be in the turned-off state, for switching the molecules for generating effector molecules controllable by the molecular switch from the turned-off state to the turned-on state, for switching the molecules for generating effector molecules controllable by the molecular switch from the turned-on state to the turned-off state, and the like. For example, the effector molecules controllable by the molecular switch under the condition of illumination by the second light can be enabled to be in the turned-on state. As another example, effector molecules controllable by the molecular switch under the condition of simultaneous illumination by the first light and the second light can be enabled to be in the turned-off state. As yet another example, the effector molecules controllable by the molecular switch under the condition of illumination by the first light can be enabled to be in the turned-off state. As still another example, the condition of illumination by the first light can generally be different from the condition of illumination by the second light.

In the lithographic method, the partial overlap of the first light and the second light can be such that only part of the first light overlaps with only part of the second light, or such that one light in its entirety overlaps with part of the other light. For example, the first light in its entirety can overlap with part of the second light. As another example, the second light in its entirety can overlap with part of the first light.

In the lithographic method, the first light can be single hollow light or multiple hollow light, and the second light can at least partially or completely cover a non-illuminated area surrounded by an area illuminated by the first light. A hollow light typically refers to any light that can form an illuminated area surrounding a non-illuminated area that is significantly less illuminated than the illuminated area, with almost or exactly no illumination in the non-illuminated area. There can be one or more non-illuminated area , the first light can be a single hollow light when there is one non-illuminated area surrounded by the illuminated area formed by the first light, and the first light can be multiple hollow light when there are multiple non-illuminated areas surrounded by the illuminated area formed by the first light. The non-illuminated area surrounded by the illuminated area formed by the first light can be of various regular or irregular shapes, and the shapes can be on a nanoscale in a certain dimension. For example, in an embodiment of the present application, the shape of the non-illuminated area can be a circle, an ellipse, a polygon, an extending line, and the like. The diameter of the circle, the major axis of the ellipse and the minor axis of the ellipse, the diameter of the polygon, the width of the extending line, and the like can be not greater than 5nm, 5-10nm, 10-15nm, 15-20nm, 20-25nm, 25-30nm, 30-35nm, 35-40nm, 40-45nm, 45-50nm, 50-55nm, 55-60nm, 60-65nm, 65-70nm, 70-75nm, 75-80nm, 80-85nm, 85-90nm, 90-95nm, 95-100nm, 100-110nm, 110-120nm, 120-130nm, 130-140nm, 140-150nm, 150-160nm, 160-180nm, 180-200nm, or greater. In an embodiment of the present application, the hollow light can be annular light, planar light having a hollow center, and the like. Furthermore, the multiple non-illuminated areas can form an array, and the first light can itself be multiple light beams or array light. In another embodiment of the present application, the hollow light can be an annular light array, planar light having a hollow array, and the like. Planar light having a hollow array means that the multiple non-illuminated areas form an array in the illuminated area. The second light can be solid light, which generally refers to light that forms an illuminated area that does not encompass any non-illuminated areas. In some embodiments of the present application, the solid light can be a single light beam, planar light, and the like. Furthermore, the second light can be multiple light beams or array light. In another embodiment of the present application, the second light can be array light formed from a plurality of single light beams.

In the lithographic method, the area illuminated by the second light does not exceed the outer edge of the area illuminated by the first light, which generally means that the area illuminated by the second light does not include an area except the non-illuminated area surrounded by the area illuminated by the first light and the area illuminated by the first light. The illuminated areas formed by the first light and/or the second light can be illuminated areas formed by these lights on the lithographic material.

In the lithographic method, the molecules for generating effector molecules controllable by the molecular switch generally refer to a compound which can switch from the turned-on state to the turned-off state and/or from the turned-off state to the turned-on state under certain conditions, and which can generate the effector molecules under certain conditions in the turned-on state. The molecular structural formulas of the molecules for generating effector molecules controllable by the molecular switch in the turned-on state and in the turned-off state can be different. The molecules for generating effector molecules controllable by the molecular switch in the turned-on state generally means that the molecules for generating effector molecules controllable by the molecular switch can generate the effector molecules under certain conditions. The conditions under which the molecules for generating effector molecules controllable by the molecular switch in the turned-on state generate the effector molecules can be a condition of no illumination and can also include a condition of illumination, and particularly a condition of illumination by, for example, ultraviolet light, visible light, or infrared light. As another example, it can be a condition of illumination with light having a wavelength not greater than 5nm, 5-10nm, 10-20nm, 20-40nm, 40-60nm, 60-80nm, 80-100nm, 100-150nm, 150-200nm, 200-250nm, 250-300nm, 300-350nm, 350-400nm, 400-450nm, 450-500nm, 500-550nm, 550-600nm, 600-650nm, 650-700nm, 700-750nm, 750-800nm, 800-850nm, 850-900nm, 900 -950nm, 950-1000nm, 1000-1200nm, 1200-1400nm, 1400-1600nm, 1600-1800nm, 1800-2000nm, 2000-2500nm, 2500-3000nm, 3000-3500nm, 3500-4000nm, 4000-4500nm, 4500-5000nm, 5000-6000nm, 6000-7000nm, 7000-8000nm, 8000-9000nm, 8000-9000nm, 8000-9000nm, 8000-9000nm, 8000-9000nm, 8000-9000nm, 9000-10000nm, 10000-12000nm, 12000-14000nm, 14000-16000nm, 16000-18000nm, 18000-20000nm, or not less than 20000nm. The molecules for generating effector molecules controllable by the molecular switch in the turned-off state generally means that the molecules for generating effector molecules controllable by the molecular switch in the turned-off state can hardly generate effector molecules under the same or similar conditions with respect to the molecules for generating effector molecules controllable by the molecular switch in the turned-on state. In some embodiments of the present application, the molecules for generating effector molecules controllable by the molecular switch generate the effector molecules by breaking chemical bonds of some groups of the molecules for generating effector molecules controllable by the molecular switch, and by converting the molecules for generating effector molecules controllable by the molecular switch into the effector molecules because of changes in groups thereof.

In the lithographic method, at least part or all of the molecules for generating effector molecules controllable by the molecular switch in the turned-on state can generate the effector molecules, and a person skilled in the art would be able to select a suitable compound sensitive to the effector molecules according to the type of the effector molecules to form a lithographic material containing the compound sensitive to the effector molecules, so that the effector molecules can change the physical properties and/or chemical properties of the lithographic material in the area where the molecular switch is turned on. For example, the effector molecules generated by the molecules for generating effector molecules controllable by the molecular switch can be molecules for removing protecting groups from the lithographic material, which can include but are not limited to acidic molecules, basic molecules, singlet oxygen, and the like. The compound sensitive to the effector molecules can be a polymer sensitive to the effector molecules, which can be materials including but not limited to acrylic and acrylic ester materials with protecting groups, aliphatic cyclic olefin materials, maleic anhydride materials, and the like. A person skilled in the art can select an appropriate group as a protecting group of the compound sensitive to the effector molecules according to parameters such as the type of the effector molecules, the reaction conditions, and the like. The protecting group can include but is not limited to t-BOC and the like. More specifically, the protecting group in the molecular structure of the compound sensitive to the effector molecule can be removed in the presence of the molecules for removing protecting groups from the lithographic material so that the physical and/or chemical properties of the deprotected lithographic material (relative to the non-deprotected lithographic material) can be changed. The change of the physical and/or chemical properties of the lithographic material can generally be a change in the solubility of the lithographic material in a developing solution, for example. The protecting group in the molecular structure of the compound sensitive to the effector molecule can be removed in the presence of the molecules for removing protecting groups from the lithographic material so that the solubility of the deprotected lithographic material in the developing solution can be increased or decreased. As another example, the effector molecules generated by the molecules for generating effector molecules controllable by the molecular switch can be a lithographic material dissolution inhibitor, which can be a material including but not limited to diazonaphthoquinones, and the like, and the compound sensitive to the effector molecules can be a polymer sensitive to the effector molecules, which can be materials including but not limited to novolac and the like. A person skilled in the art can select an appropriate polymer sensitive to the effector molecules as the compound sensitive to the effector molecules according to parameters such as the type of the effector molecules, reaction conditions, and the like. The polymer sensitive to the effector molecules can be a material including but not limited to phenolic aldehyde and the like. More specifically, physical properties and/or chemical properties of the compound sensitive to the effector molecules can be changed in the presence of a lithographic material dissolution inhibitor. The change of the physical and/or chemical properties of the lithographic material can generally be a change in the solubility of the lithographic material in the developing solution. For example, the solubility of the lithographic material in the developing solution can be increased or decreased in the presence of the lithographic material dissolution inhibitor. As another example, the effector molecules generated by the molecules for generating effector molecules controllable by the molecular switch can be molecules for activating polymerization control of the lithographic material, and more specifically they can be polymerization initiation molecules. The molecules for activating polymerization control of the lithographic material can include but are not limited to acidic molecules, basic molecules, singlet oxygen, various polymerization initiators, and the like. The compound sensitive to the effector molecules can be an polymerized monomer or oligomer sensitive to the effector molecules and the like. The molecules for activating polymerization control of the lithographic material can be the same as the polymerized monomer or oligomer sensitive to the effector molecules. The compound sensitive to the effector molecules is generally a monomer and/or oligomer and the like that can undergo a polymerization reaction. More specifically, the compound sensitive to the effector molecules can include but is not limited to an acrylate-based molecular monomer, a methacrylate-based molecular monomer, a vinyl-based molecular monomer, a vinyl ether-based molecular monomer, an epoxy-based molecular monomer, and the like. The monomers or oligomers in the lithographic material can undergo polymerization in the presence of the molecules for activating polymerization control of the lithographic material, thereby changing the physical and/or chemical properties of the lithographic material. The change of the physical and/or chemical properties of the lithographic material can generally be a change in the solubility of the lithographic material in the developing solution. For example, the monomer or oligomer as the compound sensitive to the effector molecules can undergo polymerization in the presence of the molecules for activating polymerization control of the lithographic material, thereby increasing or decreasing the solubility of the polymerized lithographic material in the developing solution.

In the lithographic method, a molecular structure of the molecule for generating effector molecules controllable by the molecular switch can contain a molecular switch group and an effector molecule generating group. The molecule for generating effector molecules controllable by the molecular switch containing the molecular switch group can generally change in accordance with certain conditions so that the molecules for generating effector molecules controllable by the molecular switch can be switched from the turned-on state to the turned-off state and/or from the turned-off state to the turned-on state (the conditions for enabling the molecules for generating effector molecules controllable by the molecular switch to be in the turned-off state can include the condition of first light illumination; and the conditions for enabling the molecules for generating effector molecules controllable by the molecular switch to be in the turned-on state can include a condition of second light illumination). For example, a substance having the molecular switch group can be a substance having structures including but not limited to proton transfer tautomerism, cis-trans isomerism, bond heterolysis, a pericyclic reaction system, and the like, and more specifically it can be compounds including but not limited to diarylethenes, azobenzenes, spiropyrans, spirooxazines, fulgides, salicylaldehyde aniline Schiff base, and the like. The molecules for generating effector molecules controllable by the molecular switch containing the effector molecule generating group can release the effector molecules under certain conditions when the molecules for generating effector molecules controllable by the molecular switch are in the turned-on state (the conditions under which the effector molecules are released can include the condition of light illumination under which the effector molecules are generated as described above). The molecules for generating effector molecules controllable by the molecular switch capable of generating the effector molecules can also undergo a conformational conversion under certain conditions when the molecules for generating effector molecules controllable by the molecular switch are in the turned-on state (the conditions of conversion to the effector molecule can include the condition of light illumination under which the effector molecules are generated as described above) so that the molecule for generating effector molecules controllable by the molecular switch in the turned-on state converts itself to the effector molecule. The substances having the effector molecule generating group can include but are not limited to photoacid generating molecules, photobase generating molecules, photosensitive molecules, polymerization initiator generating molecules, and the like, so that acidic molecules, basic molecules, singlet oxygen, polymer initiators, and the like can be released as the effector molecules, respectively.

The photoacid generating molecules can include ionic and nonionic types, wherein the ionic type can specifically include but is not limited to diazohydrochloride compounds, diazosulfate compounds, diazosulfonate compounds, diazofluorophosphate compounds, onium salt compounds, and the like. The onium salt compounds can include but are not limited to iodonium salt compounds, selenonium salt compounds, phosphonium salt compounds, arsonium salt compounds, and the like. The non-ionic type of groups can include but are not limited to polyhaloacetophenone compounds, triazine derivative compounds, sulfonyl chloride ester compounds, and the like. The photobase generating molecules can include but are not limited to transition metal ion ammonia complex compounds, quaternary ammonium salt compounds, ester compounds (including ketoxime ester compounds, carbamate compounds, carbamate oxime ester compounds), formamide compounds, triaryl carbinol compounds, and the like. The photosensitive molecules can be porphyrin compounds or phthalocyanine molecule compounds. The polymerization initiator generating molecules can be benzoin ether compounds, benzil ketal compounds, acetophenone compounds, acyl phosphine oxide compounds, α-chloroacetophenone compounds, sulfonyl acetophenone compounds, sulfonyl oxyacetophenone compounds, azo compounds, peroxy (thio) compounds, benzophenone compounds, thioxanthone compounds, quinone compounds, diazonium salt compounds, onium salt compounds, and the like. In the molecule for generating effector molecules controllable by the molecular switch, the molecular switch group and the effector molecule generating group can be connected by a chemical bond, which can be, for example, an ionic bond, a covalent bond, and the like. In the embodiments of the present application, the molecules for generating effector molecules controllable by the molecular switch can be p-toluenesulfonic acid compounds, trifluoromethanesulfonic acid compounds, methanesulfonic acid compounds, p-toluenesulfonic acid sulfonium salt compounds, trifluoromethanesulfonic acid sulfonium salt compounds, methanesulfonic acid sulfonium salt compounds, and the like.

In the lithographic method, since the molecules for generating effector molecules controllable by the molecular switch in the turned-on state can be enabled to generate the effector molecules under conditions including light illumination, the methods for enabling the effector molecules controllable by the molecular switch in the turned-on state to generate the effector molecules can include changing the condition of light illumination to which the molecules for generating effector molecules controllable by the molecular switch in the turned-on state are subjected. For example, a third light beam can be provided to the molecules for generating effector molecules controllable by the molecular switch in the turned-on state, or parameters of the second light beam (e.g., illumination intensity, wavelength and the like) can be changed such that the molecules for generating effector molecules controllable by the molecular switch in the turned-on state generate the effector molecules.

The lithographic method provided by the present application can further comprise removing either the lithographic material that has changed in physical or chemical properties or the lithographic material that has not changed. A person skilled in the art can select an appropriate method for removing the lithographic material that has changed in physical or chemical properties or the lithographic material that has not changed according to the type of the lithographic material. For example, a portion of the lithographic material that is more soluble in the developing solution can be removed by dissolving the lithographic material in the developing solution.

### Compound

Another aspect of the present application provides a compound that is the molecules for generating effector molecules controllable by the molecular switch as described above.

In the compound provided herein, the effector molecules produced by the compound can be molecules for removing protecting groups from the lithographic material, which can include but are not limited to acidic molecules, basic molecules, singlet oxygen, and the like. The effector molecules generated by the molecules produced by the compound can be lithographic material dissolution inhibitors, which can include but are not limited to diazonaphthoquinone and the like. The effector molecules generated by the molecules for generating effector molecules controllable by the molecular switch can also be molecules for activating polymerization control of the lithographic material, and more specifically can be polymerization initiation molecules, which can include but are not limited to acidic molecules, basic molecules, singlet oxygen, polymerization initiators, and the like.

### Lithographic material

Another aspect of the present application provides a lithographic material comprising the molecules for generating effector molecules controllable by the molecular switch and a compound sensitive to the effector molecules.

In the lithographic material provided by the present application, the compound sensitive to the effector molecules can be a polymer sensitive to the effector molecules, which can be materials including but not limited to acrylic and acrylic ester materials with protecting groups, aliphatic cyclic olefin materials, maleic anhydride materials, and the like. A person skilled in the art can select an appropriate group as a protecting group of the compound sensitive to the effector molecules according to parameters such as the type of the effector molecules, the reaction conditions, and the like, and the protecting group can include but is not limited to t-BOC and the like. More specifically, the protecting group in the molecular structure of the compound sensitive to the effector molecule can be removed in the presence of the molecules for removing protecting groups from the lithographic material so that the physical and/or chemical properties of the deprotected lithographic material (relative to the non-deprotected lithographic material) can thus be changed. In comparison with the compound sensitive to the effector molecules without being deprotected, the solubility of the deprotected compound sensitive to the effector molecules in a developing solution is generally different. For example, the solubility of the deprotected compound sensitive to the effector molecules increases or decreases in the developing solution. The compound sensitive to the effector molecules can be materials such as novolac, and more specifically can be materials including but not limited to novolac, formaldehyde, and the like. The physical and/or chemical properties of the compound sensitive to the effector molecules can be changed in the presence of the lithographic material dissolution inhibitor. The change of the physical and/or chemical properties of the lithographic material can generally be a change in the solubility of the lithographic material in the developing solution. For example, the solubility of the lithographic material in the developing solution can be increased or decreased in the presence of the lithographic material dissolution inhibitor. The compound sensitive to the effector molecules can be a polymerized monomer or oligomer sensitive to the effector molecules or the like, and the compound sensitive to the effector molecules is generally a polymerizable monomer and/or oligomer or the like. More specifically, the compound sensitive to the effector molecules can include but is not limited to an acrylate-based molecular monomer, a methacrylate-based molecular monomer, a vinyl-based molecular monomer, a vinyl ether-based molecular monomer, an epoxy-based molecular monomer, and the like. The monomers or oligomers in the lithographic material can undergo polymerization in the presence of the molecules for activating polymerization control of the lithographic material, thereby changing the physical and/or chemical properties of the lithographic material. In comparison with the non-polymerized lithographic material, the resulting material after the polymerization in the developing solution is different. For example, the solubility of the polymerized lithographic material in the developing solution increases or decreases.

Also included in the lithographic material provided herein can be components that can be included in various other lithographic materials, such as catalysts, initiators, adjuvants, and the like. The adjuvants can be various antifoaming agents, leveling agents, stabilizers, dispersants, and the like that are suitable in the art for lithographic materials.

The lithographic method and the lithographic material provided herein are based on a dual-beam super-resolution technology. A semiconductor laser, which is a mature technology, is adopted as the light source. Costs are low, the resolution has no theoretical limit and can reach a process node below 10nm, and the costs for high-resolution lithography are greatly reduced. The details of the advantages are as follows.
1. The existing laser direct-writing technology mainly adopts a semiconductor laser. The resolution of a semiconductor laser cannot break through to a level below 100nm due to the limitation of the wavelength of the laser. As a result, processing below 100nm cannot be realized by the current common laser direct-writing technology, and assistance of a higher resolution method such as an electron light beam is still necessary. The present application gets rid of the limitation of the wavelength on the basis of the dual-beam principle and thus can realize resolutions below 10nm.
2. In comparison with technologies based on a maskless super-resolution lithography process such as electron light beam direct writing and the like, the present application adopts a solid-state semiconductor laser with low costs, the luminous efficiency is high, a high-voltage power supply is not necessary, and the cost of a single light source is low so that expansion to a plurality of light sources can be facilitated. Moreover, it is easier to render multiple light beams by this method than to render multiple light beams by adopting an electron light beam. Therefore, the present invention is more likely to render large-scale, large-range and large-breadth super-resolution nanometer multi-light beam lithography at a significantly improved speed compared with an electron light beam.
3.In comparison with lithography technology based on the near-field super resolution principle, the super resolution lithography realized by the dual-beam principle adopted in the present application is a far-field technique, but it usually requires that the imaging physical distance be controlled within the near-field range, namely within a wavelength, to realize near-field super resolution, which is very difficult in actual lithography. Moreover, if Super-RENS (Super Resolution Near-field Structure), namely the lithography of the near-field super-resolution structure process is used, it is necessary, in the first place, to realize a super-RENS layer on an upper layer of photoresist which features a more complex process and very high costs. Instead, the far-field dual-beam super-resolution realized by the present application enables PSPAG to be directly chemically reacted on the photoresist by means of dual beams in the far-field range without being limited by the physical distance of near-field imaging. The imaging is simple and feasible, and the photoresist process is completely consistent with the current mainstream process, which is mature and features good bonding properties.

Photoacid generation of the conventional positive lithographic PAG is not controllable itself, and the spatial resolution of chemical amplification etching after photoacid generation is regulated by the wavelength of a light source and the numerical aperture of a lens. The PAG designed in the invention can realize controllable photoacid release under the control of the molecular switch, thereby effectively realizing spatial-resolution regulation and control of photoacid release by using a dual-beam photo-reversible regulation and control mode, and eliminating the limitations of the light source wavelength and the numerical aperture.

Below, embodiments of the present invention are described through specific examples. Those skilled in the art can easily understand other advantages and effects of the present invention from the content disclosed in the description. The present invention can also be implemented or applied through different embodiments, and various details in the description can also be modified or changed on the basis of different viewpoints and applications without departing from the spirit of the present invention.

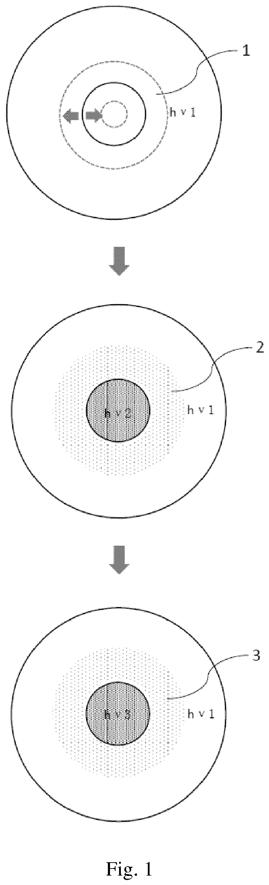

The process depicted in FIG. 1 is a process diagram of basic principles for implementing super-resolution using a dual-beam technology of the present invention. Step 1 in the figure is to turn on the annular inhibiting light 1, namely, hv1, which is formed by phase conversion. The annular hollow thereof can be adjusted in size, and therefore the resolution of the dual beams can be adjusted by adjusting the size of the hollow part of the annular inhibiting light 1. In the present invention, the light beam 1 functions to turn off the molecular switch (even if the molecules for generating effector molecules controllable by the molecular switch are in the turned-off state). Step 2 in the figure is to combine light beam 2, i.e., hv2, concentrically upon the annular light 1. Annular light 2 is a Gaussian activating light beam and functions in the present invention to turn on the molecular switch (even if the molecules for generating effector molecules controllable by the molecular switch are in the turned-on state). Step 3 of the dual-beam process provided by the invention is to excite a photochemical reaction when the molecular switch is turned on. In one method, hv3 of another wavelength can be directly combined not by adjusting the annular inhibiting light 1 with light 1 and light 2 being turned either on or off, and after the third wavelength directly acts on a photochemical group controlled by the molecular switch, only the turned-on part of the molecular switch generates photochemical effects. In another method, the photochemical group has the same action wavelength as the molecular switch, and the intensity of light 1 and light 2 should be increased synchronously to reach the threshold of photochemical group reaction.

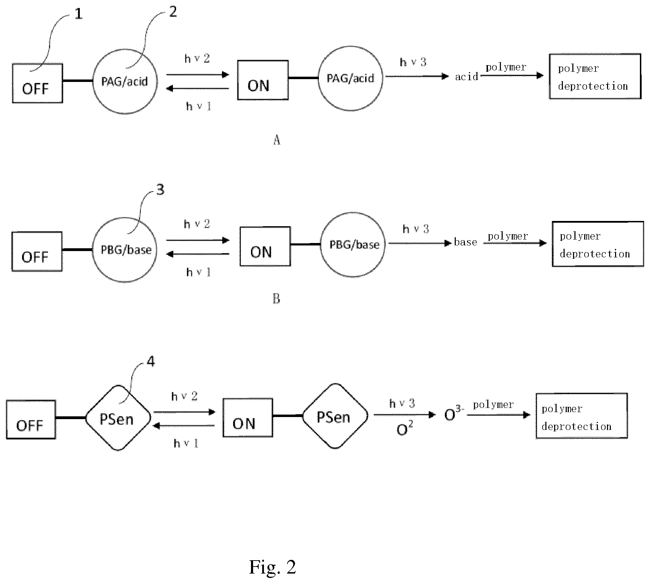

FIG. 2 is diagrams of patterns of polymerization deprotection controllable by the molecular switch. As shown in FIG. 2, the molecular characteristics and reaction patterns of several types of polymer deprotection which are finally realized under the control of the molecular switch are described, and the finally realized form is positive lithography. In FIG. 2, reference numeral 1 denotes a moiety having a molecular switch function (hereinafter referred to as molecular switch 1), reference numeral 2 denotes a moiety having a photoacid generating molecular function or a strong acid group (hereinafter referred to as acid generating group 2), reference numeral 3 denotes a moiety having a photobase generating agent function or a base molecular group (hereinafter referred to as base generating group 3), and reference numeral 4 denotes a moiety having a photosensitizer molecular function (hereinafter referred to as photosensitive molecule 4). Diagram A shows a photoacid generating molecule controllable by the molecular switch (PSPAG), wherein features of the photoacid generating molecule include two forms which are a photoacid generating agent molecules and strong acid groups. The molecular switch 1 can switch the compound from an inactive OFF state to an active ON state under the illumination of the activating light beam hv2 or from the active ON state to the inactive OFF state under the illumination of the annular inhibiting light beam hv1. In addition, the molecular switch 1 can realize repeated switching under the two types of light hv1 and hv2 in alternation. For the PSPAG molecule described in diagram A, when the compound is in the OFF state, the acid generating group 2 cannot release strong acid molecules or the strong acid molecule group cannot be dissociated from the compound, and when the compound is switched to the ON state, the acid generating group 2 releases the strong acid molecules under the illumination of hv3 or the strong acid group is dissociated from the compound. The released strong acid molecules react with specific protecting groups of the resin material to dissociate the protecting groups so as to deprotect the resin molecules. Diagram B shows a photobase generating molecule controllable by the molecular switch (PSPBG), wherein features of the photobase generating molecular include two forms which are photobase generating agent molecules and strong base groups. For the PSPBG molecule described in diagram B, when the compound is in the OFF state, the base generating group 3 cannot release strong acid molecules or the strong acid molecule group cannot be dissociated from the compound, and the compound is switched from the inactive OFF state to the active ON state under the illumination of an activating light beam hv2, and the base generating group 3 releases strong base molecules under the illumination of hv3 or the strong base group is dissociated from the compound. The released strong base molecules react with specific protecting groups of the resin material to dissociate protecting groups so as to deprotect the resin molecules. Diagram C shows a photosensitive molecule controllable by the molecular switch (PSPSen). The activating light beam hv2 switches the compound from the OFF state to the ON state, whereby the photosensitizer molecule 4 is activated for photosensitive activation, and the annular light beam hv1 switches the photosensitive molecule 4 off for photosensitive activation. In diagram C, the activated photosensitive molecule 4 activates the oxygen molecule into singlet oxygen O3- under hv3 at a specific wavelength. The singlet oxygen has strong oxidizability and reacts with specific protecting groups of the resin material to dissociate the singlet oxygen so as to realize deprotection.

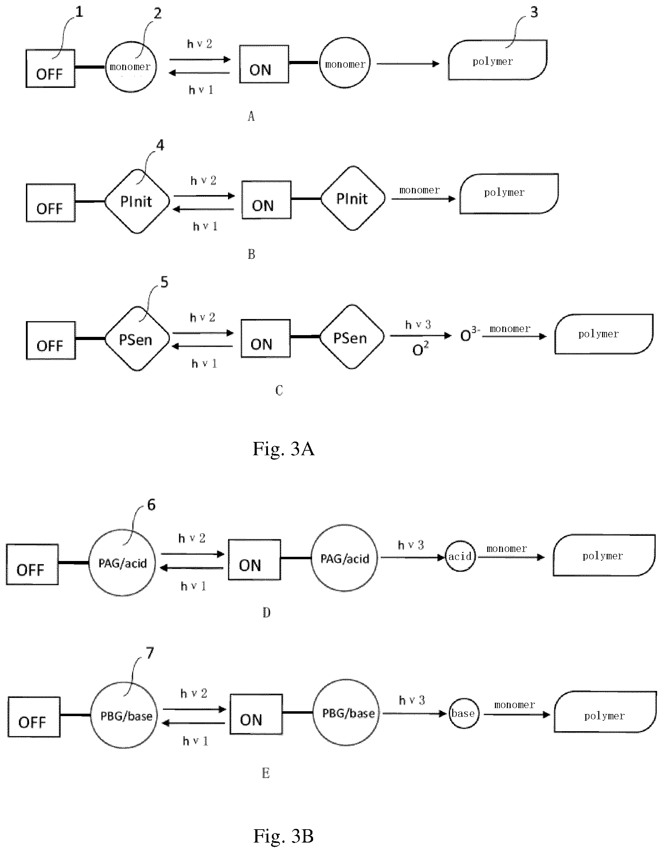

FIGS. 3A and 3B are diagrams of patterns of polymerization of monomers controllable by the molecular switch. FIGS. 3A and 3B show the molecular characteristics and reaction patterns of polymerization of polymer monomer molecules controllable by the molecular switch. In FIGS. 3A and 3B, reference numeral 1 denotes a moiety having a molecular switch function (hereinafter referred to as molecular switch 1), reference numeral 2 denotes a monomer molecule, reference numeral 3 denotes a polymerized molecule, reference numeral 4 denotes a moiety having a photoinitiator molecule function, reference numeral 5 denotes a moiety having a photosensitizer molecule function (hereinafter referred to as photosensitizer molecule 5). Reference numeral 6 denotes a moiety having a photoacid generating molecule function (hereinafter referred to as photoacid generating molecule 6), and reference numeral 7 denotes a moiety having a photobase generating molecule function (hereinafter referred to as photobase generating molecule 7). In FIGS. 3A and 3B, diagram A shows the polymer monomer molecules controllable by the molecular switch. The compound can be switched from the inactive OFF state to the active ON state under the illumination of the activating light beam hv2 and from the active ON state to the inactive OFF state under the illumination of the annular inhibiting light beam hv1. The compound can be switched on and off repeatedly under hv1 and hv2 in alternation. For the polymer monomer molecules controllable by the molecular switch shown in diagram A in FIGS. 3A and 3B, when the compound is in the OFF state, the monomer molecule 2 cannot be polymerized, when the compound is switched to the ON state by the activating light beam hv2, the monomer molecule 2 can be polymerized, and after the activation, the monomer molecule 2 can be polymerized under the combined action of illumination of hv3 and other components to generate the polymer 3. In FIGS. 3A and 3B, diagram B shows the photoinitiator molecule controllable by the molecular switch (the PInit molecule 4). This molecule, which is in the inactive OFF state, cannot initiate polymerization, and upon the action of the activating light beam, the molecular switch activates PInit, thereby initiating polymerization under the action of the effector light hv3 and the monomer molecule. In FIGS. 3A and 3B, diagram C shows the photosensitive molecule controllable by the molecular switch (PSPSen). The activating light beam hv2 switches the compound from the OFF state to the ON state, whereby the photosensitizer molecule 5 is activated for photosensitive activation, and the annular light beam hv1 switches the photosensitizer molecule 5 off for photosensitive activation. In diagram C, the activated PInit molecule 4 activates the oxygen molecule into singlet oxygen O3- under hv3 at a specific wavelength, and the singlet oxygen initiates polymerization of specific monomer molecules to effect molecular polymerization. In FIGS. 3A and 3B, diagram D shows a photoacid generating molecule controllable by the molecular switch (PSPAG). The photoacid generating group 6 can be repeatedly switched on and off under hv 1 and hv2 in alternation. The activated photoacid generating molecule 6 releases a strong acid molecule under specific illumination of hv3 and polymerizes the monomer under the combined action of other components. Diagram E shows a photobase generating molecule controllable by the molecular switch (PSPBG). The photobase generating group 7 is activated by hv2 to release a strong base molecule under specific illumination of hv3 and polymerizes the monomer under the combined action of other components.

An optically controlled molecular switch generally means that molecules can be reversibly converted to different molecular conformations under different wavelengths of light, thereby exhibiting switch characteristics. At present, the reaction forms of molecular switches can be divided into proton transfer tautomerism, cis-trans isomerism, bond heterolysis, pericyclic reaction systems, and so on.

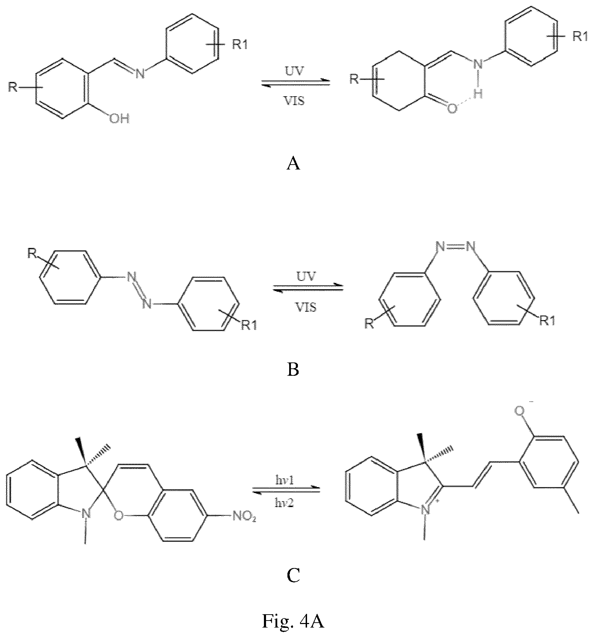

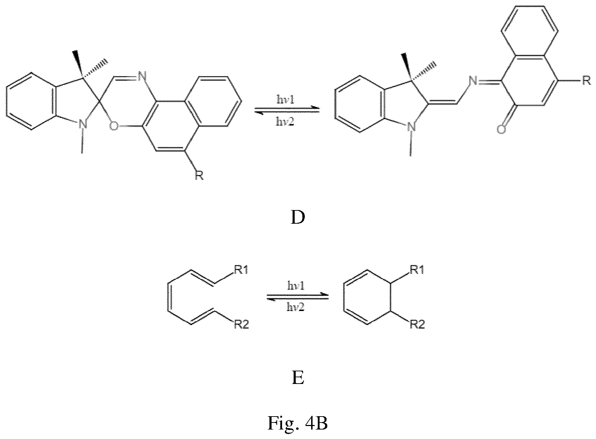

Molecules with switch characteristics are mainly concentrated in diaryl ethylene compounds, azobenzene compounds, spiropyran compounds, spirooxazine compounds, fulgide compounds, and the like. According to the invention, the molecular switch includes the above reaction forms of molecular switches. As shown in FIGS. 4A and 4B, A is a salicylaldehyde aniline Schiff base compound in a proton transfer tautomeric form, and the molecular switch conversion can be realized under UV/VIS. In FIGS. 4A and 4B, B is a cis-trans isomeric form, and azobenzenes are a class of molecules having excellent switch characteristics. In FIGS. 4A and 4B, C and D are spiropyran compounds and spirooxazine compounds in a heterolytic reaction form of a bond. Both spiropyran compounds and spirooxazine compounds can achieve excellent molecular switch performance under two different switching lights. In FIGS. 4A and 4B, E is a pericyclic reaction system, which is characterized in that a hexatriene structure in a molecule is switched to a cyclohexadiene structure under illumination, and reverse switching can be rendered under illumination. Switch molecules meeting such characteristics include fulgides, diarylethenes, and the like.

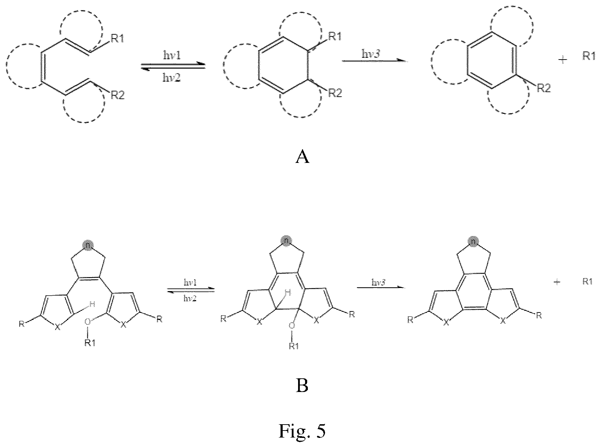

FIG. 5 shows a group-removing reaction pattern of the molecular switch of the pericyclic reaction system FIG. 5 shows a molecular switch based on the molecular switch of the pericyclic reaction system to control photoacid\photobase group removal. Photoacid generating agents are mainly divided into the ionic type and the nonionic type. In the present invention, the ionic photoacid generating agents mainly include diazonium salt compounds such as diazonium hydrochloride compounds, diazonium sulfate compounds, diazonium sulfonate compounds, and diazofluorophosphate compounds, and onium salt compounds such as iodonium salt compounds, selenonium salt compounds, phosphonium salt compounds, arsonium salt compounds, and the like. The non-ionic photoacid generating agents mainly include organic halogenated compounds such as polyhalogenated acetophenones, triazine derivatives, sulfonyl chloride esterified compounds, and the like. As shown by A in FIG. 5, on the basis of the hexatriene structure molecule, the dotted line represents various possible ring molecule types, such as benzene ring compounds, cyclturned-ontane compounds, cyclturned-ontene heterocyclic compounds, and the like. The hexatriene structure molecule can reversibly interconvert to and from a cyclohexadiene structure through intramolecular ring closure under the interaction of hv1 and hv2, and the photoacid molecule at the R1 position can detach from the cyclohexadiene structure under the action of hv3, which makes the cyclohexadiene structure irreversible into a benzene ring structure. In FIG. 5, B shows a PSPAG based on a diarylethene molecular structure according to the principle of FIG. A, wherein R1 is a moiety having a function of generating a photoacid molecule, such as a strong acid group such as trifluoromethanesulfonic acid commonly used in a photoacid generating agent, only ring-closing/ring-opening reversible conversion occurs under hv1 and hv2, the strong acid group is dissociated from the molecule under the action of hv3, and diarylethenes as the molecular switch control the release of the acid group.

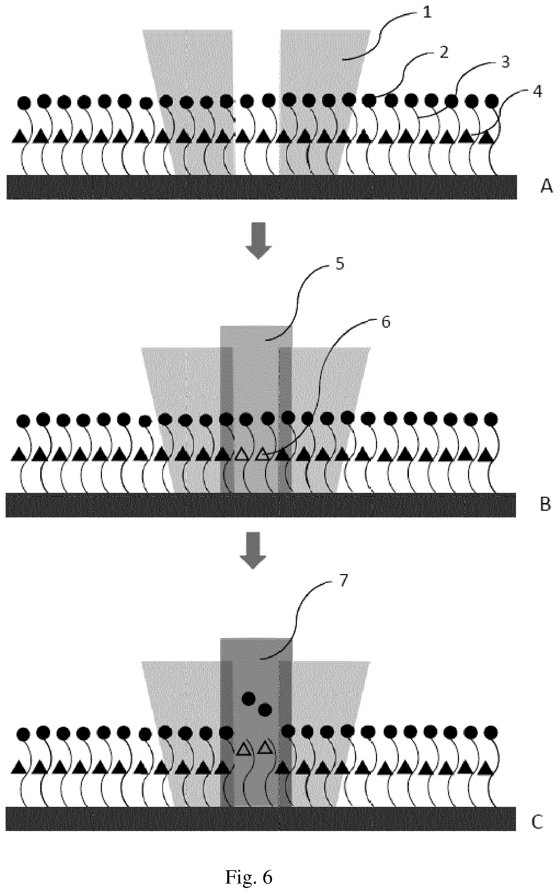

FIG. 6 is diagrams of a pattern of positive dual-beam lithography by means of polymerization deprotection controllable by the molecular switch. Namely, it shows the principle of the polymer deprotection photochemical reaction of the effector molecule controllable by the photo-molecular switch of the present invention in a photoresist under the action of dual beams. In diagram A, reference numeral 1 denotes the annular inhibiting light beam hv1, reference numeral 2 (black filled circle) denotes a protecting group carried by a photoresist macromolecule, reference numeral 3 (wavy curve) denotes a photoresist macromolecule main chain, and reference numeral 4 (black filled triangle) represents a polymer deprotection reaction molecule controllable by the molecular switch. In the process shown in diagram A, when the annular inhibiting light beam 1 illuminates the photoresist, macromolecular chains in the photoresist are all connected with protecting groups, and in the photoresist, the molecular switch controls molecules to be in the turned-off state by default so that the molecules controlled by the molecular switch do not chemically react to the photoresist when the annular light beam 1 illuminates the photoresist. In diagram B, reference numeral 5 denotes the Gaussian activating light beam hv2 concentrically combined on the annular inhibiting light beam. When the light beam is applied to the photoresist through the central aperture of the annular light beam, the protecting group carried by a photoresist macromolecule 2, which was originally in the inactive state, is activated to become what is denoted by reference numeral 6 (hollow triangle) as shown, that is, the effector molecules controlled by the molecular switches are activated. In diagram C, reference numeral 7 denotes an action light beam for switching otherwise, namely an hv3 light beam,. Under the action of the light beam 7, the activated molecule releases free effector groups, such as strong acid, strong base, singlet oxygen, and the like, which act on the protecting group 2 on the macromolecule to dissociate the protecting group 2 from the macromolecule. The dissociated photoresist macromolecular layer is decomposed after being exposed to the developing solution, and the protected macromolecule is not decomposed by the developing solution.

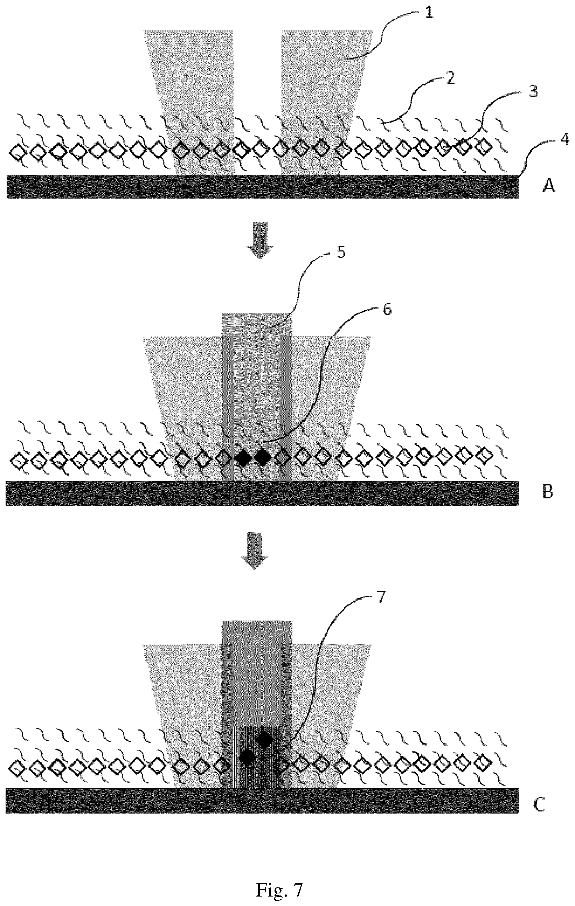

FIG. 7 is diagrams of a pattern of negative dual-beam lithography by means of monomer polymerization controllable by the molecular switch. In diagram A, reference numeral 1 denotes the annular inhibiting light beam hv1, reference numeral 2 (small wavy line) denotes a photoresist macromolecular monomer molecule, while A in FIG. 3 denotes a monomer molecule controlled by the molecular switch, reference numeral 3 (a hollow oblique square block) represents a molecular switch polymerization control molecule, and reference numeral 4 denotes a material to be treated.

In the process shown in diagram A, when the annular inhibiting light beam 1 is positioned to illuminate the photoresist, the monomer molecules in the photoresist are all in an initial depolymerization dispersion state, and in the photoresist, the molecular switch controls molecules to be in the OFF state by default, so that the molecules controlled by the molecular switch do not chemically react to the photoresist when the annular light beam 1 illuminates the photoresist. In diagram B, reference numeral 5 denotes the Gaussian activating light beam hv2 concentrically combined on the annular inhibiting light beam. When the light beam is applied to the photoresist through the central aperture of the annular light beam, either the molecular switch polymerization control molecule 3 or the photoresist macromolecular monomer molecule 2 (A in FIG. 3), which was originally in the inactive state, is activated, as shown in FIG. 6 (black solid diamond). Namely, the polymerization control molecules controlled by the molecular switches are activated. In diagram C, reference numeral 7 shows that under the action of the effector light hv3, the activated molecules initiate a polymerization reaction, whereby a curing reaction takes place at the exposure area. The pattern cured after exposure is not eluted under the treatment of a subsequent developing solution, and other unexposed areas are washed by the developing solution to reveal the surface of the material at the unexposed areas.

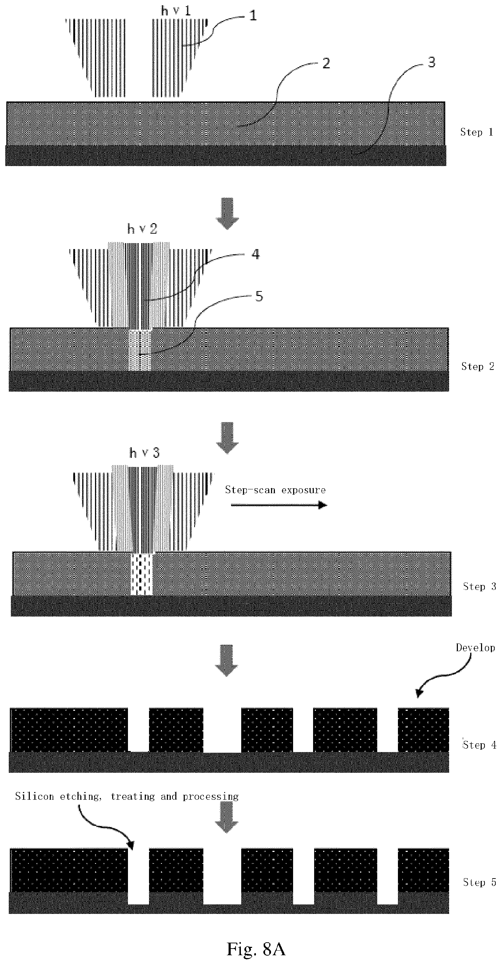

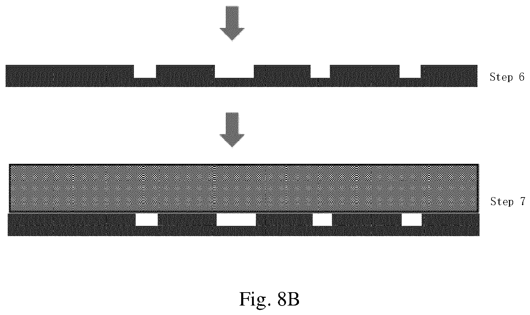

As shown in FIGS. 8A and 8B, in step 1, reference numeral 1 denotes the annular inhibiting light hv1, reference numeral 2 denotes a photoresist layer, and reference numeral 3 denotes a material to be processed, such as a silicon wafer. Taking silicon wafer lithography as an example, first, the annular inhibiting light beam is positioned on the photoresist-coated silicon wafer. In step 2, an activating light beam 4 is combined at the position where the annular light is positioned on the photoresist, namely, hv2 in FIGS. 8A and 8B, and the molecular switch effector molecules of the photoresist part 5 illuminated by the activating light beam 4 in FIGS. 8A and 8B are activated. In step 3, the effector light hv3 acts on the activated area of the photoresist, the molecular switch effector molecules in the area release effector groups, and the protecting groups in the photoresist are removed. In steps 1 to 3, step scan exposure is repeated under program control to connect and form the pattern to be etched. In step 4, after the developing solution is added, the deprotected part of the photoresist is dissolved by the developing solution, and the areas which are not exposed are not dissolved by the developing solution. In step 5, the exposed areas of the silicon wafer are subjected to etching, treatment and processing. In step 6, the photoresist in this step of operation is eluted, and the surface of the silicon wafer containing the etching pattern this time is exposed. In step 7, a new photoresist layer is applied in preparation for the next round of lithography operation.

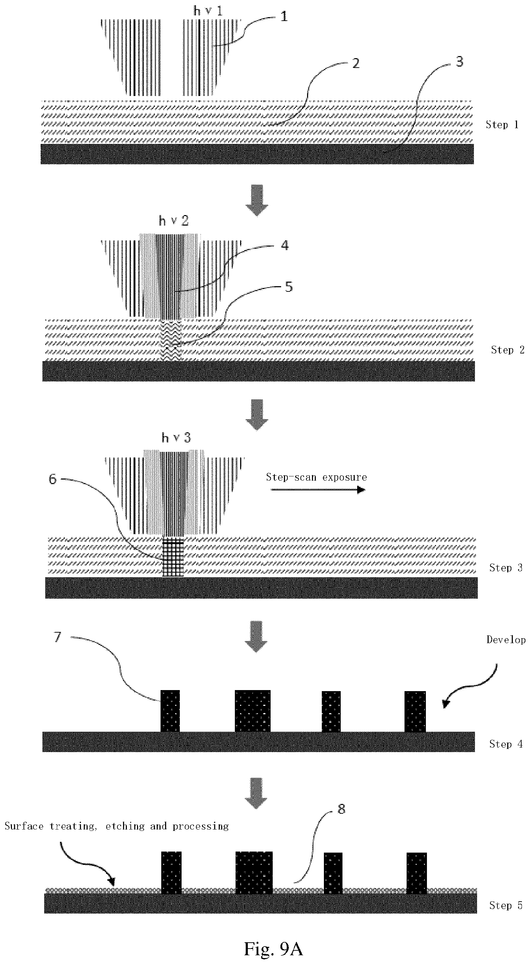

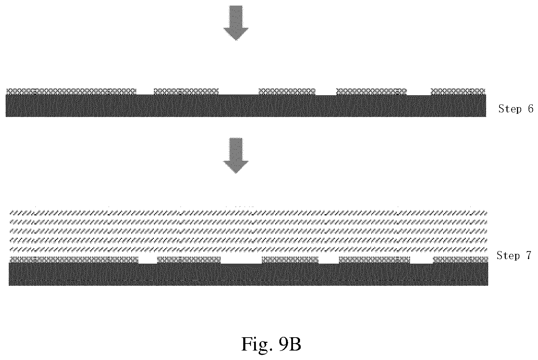

FIGS. 9A and 9B are diagrams of a process of negative dual-beam lithography by means of monomer polymerization controllable by the molecular switch. In step 1, reference numeral 1 denotes the annular inhibiting light hv1, reference numeral 2 denotes a photoresist layer, and reference numeral 3 denotes a material to be processed. First, the annular inhibiting light beam is positioned on the surface of the photoresist-coated material. In step 2, an activating light beam 4 is combined at the position where the annular light is positioned on the photoresist, namely, hv2 in FIGS. 9A and 9B, and the molecular switch polymerization control molecules of the photoresist part 5 illuminated by the activating light beam 4 in FIGS. 9A and 9B are activated. In step 3, the effector light hv3 acts on the activated area of the photoresist, and the activated polymerization control molecules in this area act with monomer components in the photoresist to initiate polymerization reaction. In steps 1 to 3, step scan exposure is repeated under program control to connect and form the pattern to be etched. In step 4, after the developing solution is added, the part of the photoresist which is not exposed is dissolved by the developing solution, and the exposed area is protected by the polymer and cannot be dissolved by the developing solution. In step 5, the exposed area of the material can be subjected to treatment, etching, processing, and the like. In step 6, the photoresist in this step is eluted to expose the surface of the material treated this time. In step 7, a new photoresist layer is applied in preparation for the next round of lithography operation.

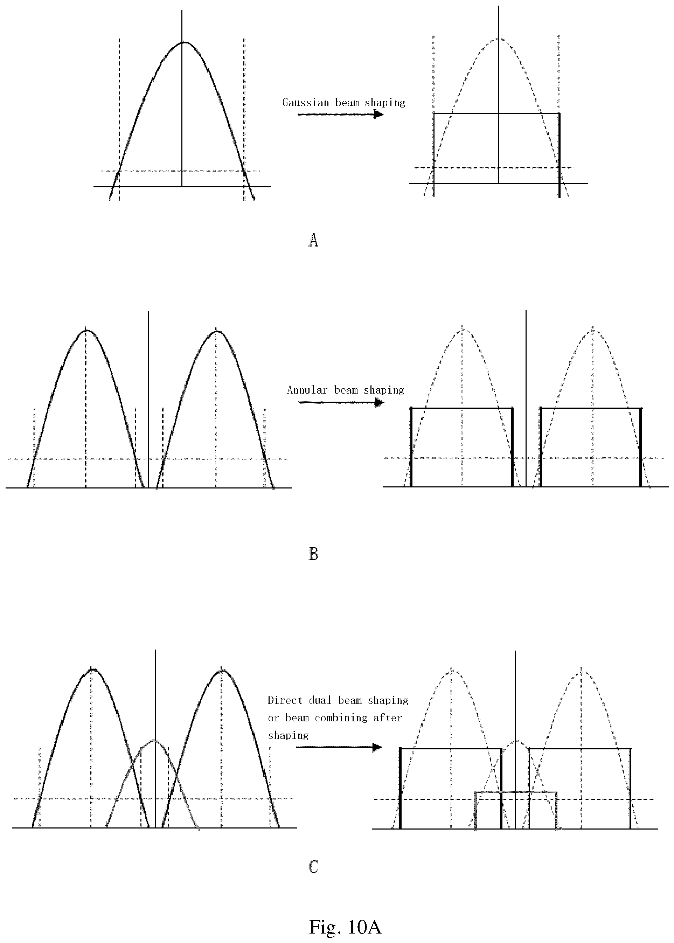

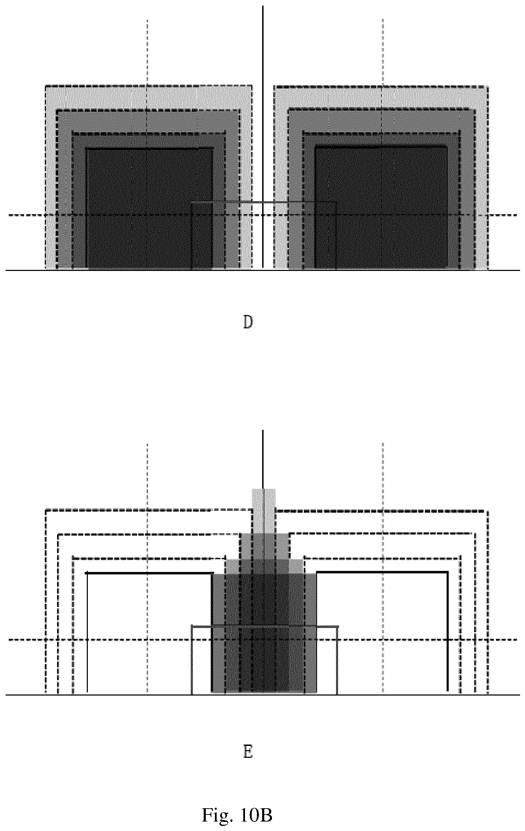

FIGS. 10A and 10B are diagrams of patterns of dual-beam shaping. A Gaussian light beam and a Gaussian annular light beam are adopted in common dual-beam systems, and the light intensity distribution is non-linear, so that non-uniform energy in an action area is produced, and the action area cannot be accurately controlled. According to the dual-beam lithography system of the present invention, a light beam shaping method is adopted, a solid light beam and a hollow light beam are modulated into a flat-top light beam form, and the control precision of a machining edge can be effectively improved. In the invention, the solid Gaussian light beams and the vortex light beams are shaped into corresponding solid flat-top light beams and flat-top dual beams by adopting a light beam shaping method, and the edges are steep and vertical, so the energy action distribution is more accurate. A in FIGS. 10A and 10B shows a solid Gaussian light beam shaped into a solid flat-top light beam. B in FIGS. 10A and 10B shows a vortex light beam shaped into a hollow flat-top light beam. C in FIGS. 10A and 10B shows that the combined dual beams can be directly shaped into flat-top dual beams, or the shaped solid flat-top light beam and the hollow flat-top light beam can be combined into a flat-top dual-beam form. D in FIGS. 10A and 10B shows that the size of the hollow is adjusted by adjusting the energy level of the hollow flat-top light beam. E in FIGS. 10A and 10B shows the final writing width of the solid flat-top light beam under different adjustments of the hollow flat-top light beam.

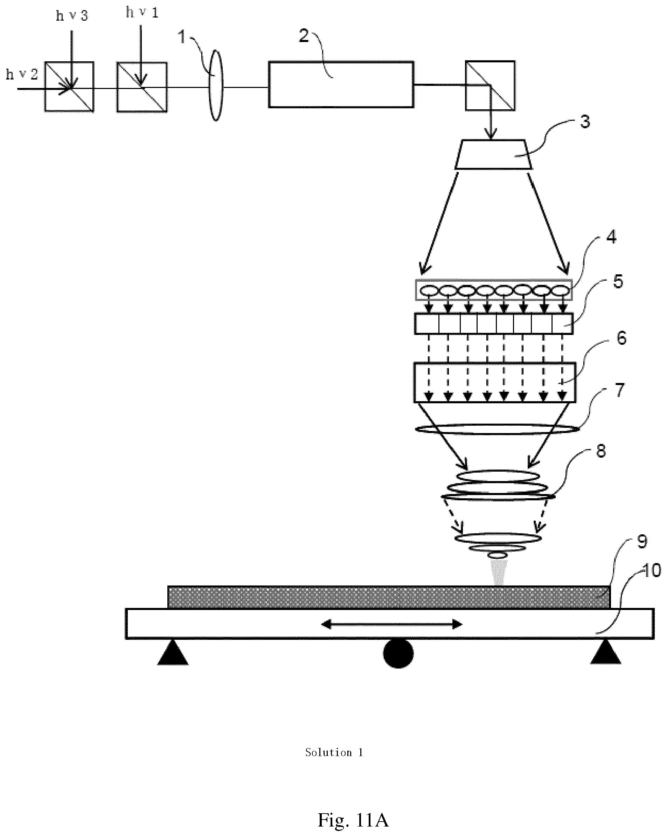

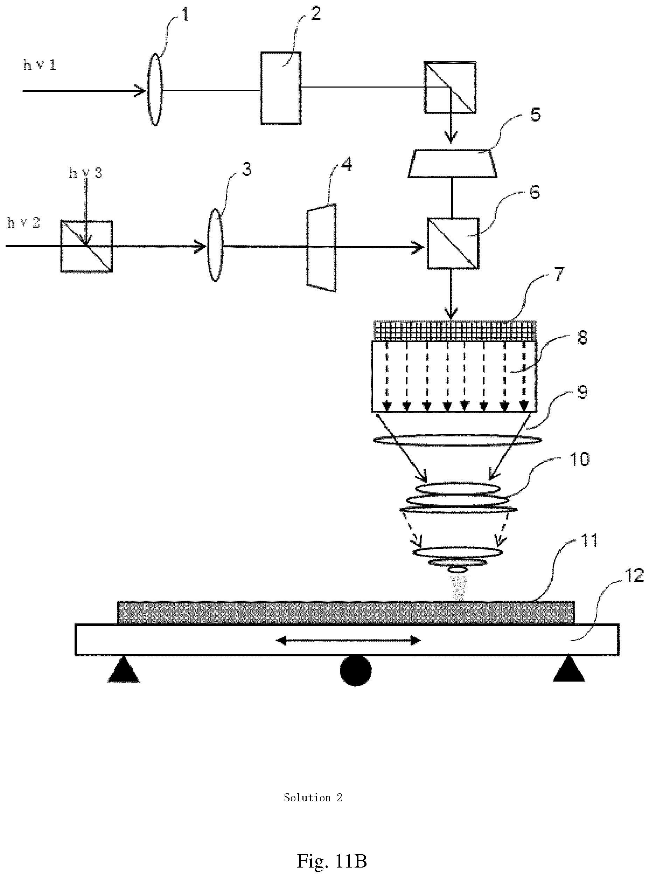

FIGS. 11A and 11B show two types of solutions of a parallelized dual-beam lithographic system. In solution 1 shown in FIGS. 11A and 11B, the dual beams are parallelized by previously directly generating concentric light beams. As shown in solution 1 of FIGS. 11A and 11B, hv1 is the initial input light beam of the annular light beam, and hv2 and hv3 are the initial input light beams of the solid Gaussian light beams. According to solution 1, hv1 and hv2 are converged through a spectroscope and then are gathered through a lens group 1 and enter a light beam shaping device 2 to realize the concentricity of hv1 and hv2. The device 2 can be generally realized by adopting a polarization maintaining fiber. The concentric light beams pass through a light beam shaping device 3 to convert the Gaussian light beam of the concentric light beams into a flat-top light beam with an even energy distribution. The converted and expanded light beams enter a microlens array group 4 and are converted into array-type multiple light beams through the microlens group. A two-light beam phase conversion array group 5 corresponds to the microlens array group 4. Each light beam coming out of the microlens array group 4 enters a corresponding phase conversion unit so that hv1 is converted from the initial light beam to the annular light beam, while hv2 remains as a solid light beam, thereby realizing arrayed dual light beams. The arrayed dual beams enter a spatial light modulator 6 corresponding to the pixel units. This device can perform on-off control of the dual beams of each pixel at a high speed by means of a computer program so that each of the dual beams can be modulated and the writing of patterns can be controlled. The dual-beam array coming out of the spatial light modulator 6 is focused through a lens group 7, then the image is miniaturized through an image-miniaturizing lens group 8, and finally high-speed parallel dual-beam lithography is realized on the surface of a lithographic material 9. The lithographic material is subjected to displacement stepping control under the control of a precision displacement platform 10.

In FIGS. 11A and 11B, solution 2 shows the use of the annular light to enable separation from the solid Gaussian light beam. As shown in solution 2 of FIGS. 11A and 11B, hv1 is the initial input light beam of the annular light beam, and hv2 and hv3 are the initial input light beams of the solid Gaussian light beams. hv1 first passes through a lens group 1 and then enters a polarizing device 2 to produce the desired polarized light beam. The solid Gaussian light beam hv2 passes through a lens group 3 and is converted into a flat-top light beam by a Gaussian light beam shaper 4. The annular light beam converted from hv1 is also converted into a hollow flat-top light beam form by an annular light beam shaper 5. After the two shaped light beams are combined through a spectroscope 6, the polarized hv1 is converted into arrayed vortex light, namely an annular light array, through a phase type diffraction grating array 7, and meanwhile the solid light beam hv2 and the annular light array are combined and jointly pass through a spatial light modulator 8. The phase type diffraction grating is coupled with the spatial light modulator. Their pixel units have a one-to-one correspondence to each other so that independent switch control of each dual-beam cell is realized, and writing of patterns is controlled. As in solution 1, the dual-beam array from the spatial light modulator 8 in solution 2 is focused through a lens group 9, the image is miniaturized through an image-miniaturizing lens group 10, and finally high-speed parallel dual-beam lithography is realized on the surface of a lithographic material 11. The lithographic material is subjected to displacement stepping control under the control of a precision displacement platform 12.

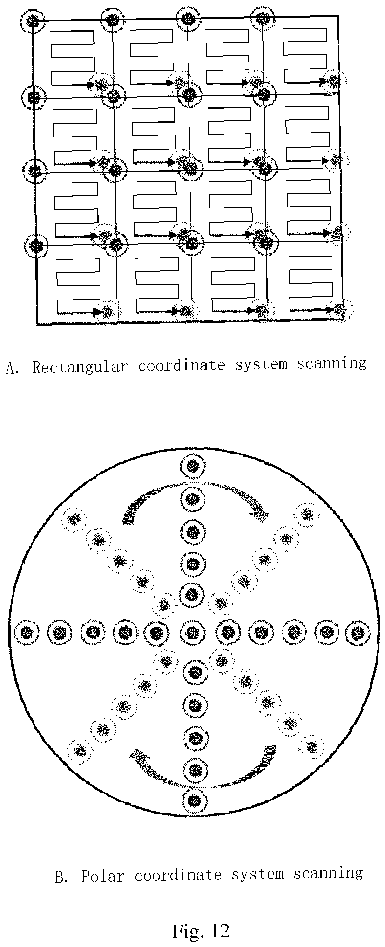

The two diagrams A and B shown in FIG. 12 show that the scanning mode adopted by the dual-beam array shown in the invention comprises a rectangular coordinate system scanning mode and a polar coordinate system scanning mode. The two coordinate system modes can switch according to different characteristics of the required lithographic pattern. In FIG. 12, diagram A shows an array scanning mode in a rectangular coordinate system in which after lithography pattern data is divided into a large number of rectangular blocks, dual beams scan from one corner vertex of the rectangular block to a diagonal vertex of the rectangular block in a snakelike mode. In FIG. 12, diagram B shows an array scanning mode in a polar coordinate system in which lithography patterns are centrosymmetrically distributed as a circle in a polar coordinate mode, and array points realize pattern lithography through circular scanning. Either of the two scanning system modes can be independently used in the lithography process, or both can be combined for interaction and switching, with a computer program controlling switching between the two coordinate systems.

### Embodiment 1

The positive lithographic method is implemented by the following steps.
1) The annular inhibiting light beam is positioned on the material coated with photoresist.
2) The solid light beam is combined on the photoresist where the annular light is positioned to carry out activation.
3) The molecular switch in the activated area of the photoresist controls an effector molecule to release the effector group, and the protecting group in the photoresist is removed. In steps 1) to 3), step scan exposure is repeated under program control, that is, to connect and form the pattern to be etched.
4) The developing solution is added to dissolve the deprotected part of the photoresist, and areas which are not exposed cannot be dissolved by the developing solution.
5) The exposed area can be subjected to etching, treatment and processing.
6) The photoresist of this round of operation is eluted to expose the surface of the material containing the etching pattern this time.
7) A new photoresist layer is applied in preparation for the next round of lithography.

### Embodiment 2

The negative lithographic method is implemented by the following steps.
1) The annular inhibiting light beam is positioned on the surface of the material coated with photoresist.
2) The activating light beam is combined on the photoresist where the annular light is positioned to control molecular activation.
3) The active polymerization control molecules in the activated area of the photoresist react with monomer components in the photoresist to initiate polymerization reaction. In steps 1) to 3), step scan exposure is repeated under program control, that is, to connect and form the pattern to be etched.
4) The developing solution is added to dissolve the unexposed part of the photoresist, and the exposed area is protected by the polymer and cannot be dissolved by the developing solution.
5) The exposed area of the material can be subjected to treatment, etching and processing, etc.
6) The photoresist in this step is eluted to expose the surface of the material treated this time.
7) Lastly, a new photoresist layer is applied in preparation for the next round of lithography.

### Embodiment 3

The dual-beam photoresist is mainly prepared from resin, a dual-beam-controllable molecular switch photosensitive acid generating agent (PSPAG), a solvent, an additive, and the like in certain proportions. The preparation method is as follows.
1) Kept out of the light, 0.005-10 parts of dual-beam-controllable molecular switch photosensitive acid generating agent (PSPAG) are dissolved in 20-90 parts of organic solvents.
2) After complete dissolution, 5-80 parts of acid degradation type resin and 0-60 parts of dissolution inhibitors are added into the solution, and the solution is stirred uniformly to obtain the dual-beam positive photoresist.

### Embodiment 4

The dual-beam photoresist is mainly prepared from resin, a dual-beam-controllable molecular switch photosensitizer (PSPSen), a photoacid generating agent, a solvent, an additive, and the like in certain proportions. The preparation method is as follows.
1) Kept out of the light, 0.0001-2 parts of dual-beam-controllable molecular switch photosensitizers (PSPSen) and 0.005-10 parts of photoacid generating agents are dissolved in 20-90 parts of organic solvents.
2) After complete dissolution, 5-80 parts of acid degradation type resin and 0-60 parts of dissolution inhibitors are added into the solution, and the solution is stirred uniformly to obtain the dual-beam positive photoresist.

### Embodiment 5

The dual-beam photoresist is mainly prepared from resin, a dual-beam-controllable molecular switch photosensitive base generating agent (PSPBG), a solvent, an additive, and the like in certain proportions. The preparation method is as follows.
1) Kept out of the light, 0.005-10 parts of dual-beam-controllable molecular switch photosensitive base generating agent (PSPBG) are dissolved in 20-90 parts of organic solvents.
2) After complete dissolution, 5-80 parts of base degradation type resin and 0-60 parts of dissolution inhibitors are added into the solution, and the solution is stirred uniformly to obtain the dual-beam positive photoresist.

### Embodiment 6

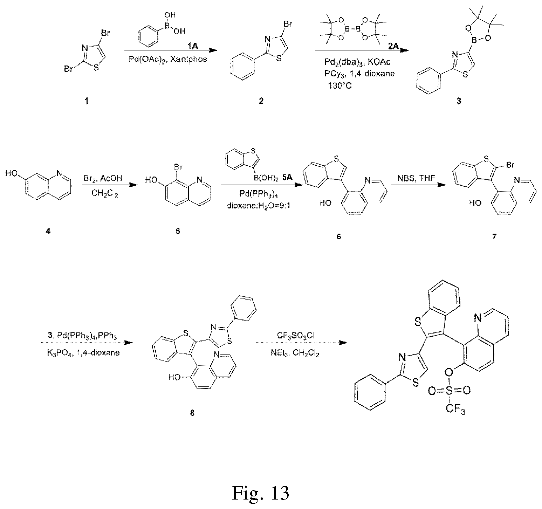

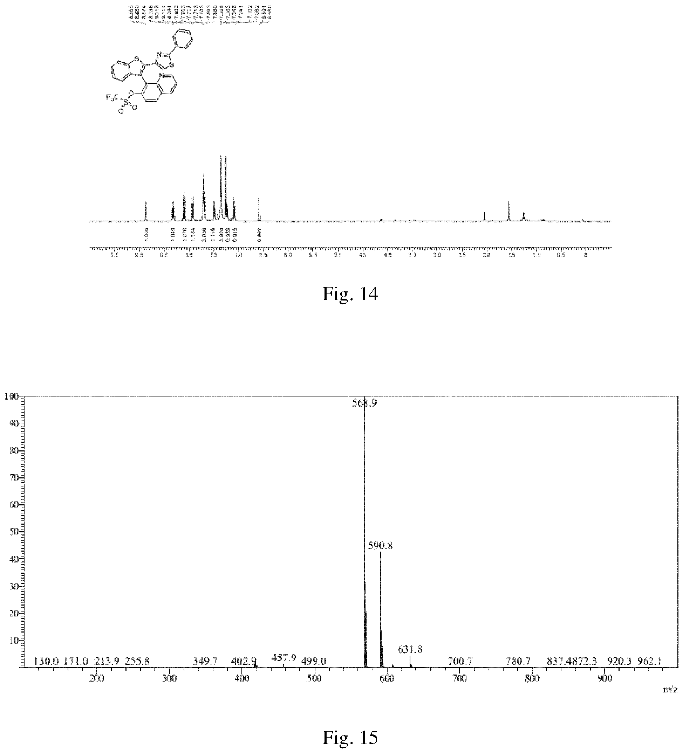

A method for synthesizing the dual-beam-controllable molecular switch photosensitive acid generating molecule (PSPAG) is as follows.
1) A synthetic route of the PSPAG molecule is shown in FIG. 13. A compound 1 is reacted with a compound 1A under the catalysis of Pd(OAc)2 and Xantphos to generate a compound 2, and compound 2 is reacted with a compound 2A under the catalysis of Pd2(dba)3, KOAc, PCy3, 1,4-dioxane at 130 to generate a compound 3.
2) A compound 4 is reacted with Br2, AcOH and CH2Cl2 to generate a compound 5, compound 5 is reacted with a compound 5A in a solvent having a ratio of dioxane to H2O of 9:1 under the catalysis of Pd(PPh3)4 to generate a compound 6,which is reacted with NBS in THF to generate a compound 7.
3) Compound 7 and compound 3 generated by the previous reaction generate a compound 8 under the catalysis of Pd(PPh3)4, PPh3, K3PO4 and 1,4-dioxane, and compound 8 is reacted with CF3SO3Cl in NEt3 and CH2Cl2 to generate a final target molecule.
4) The target molecule is finally authenticated by nuclear magnetic spectroscopy (FIG. 14) and mass spectroscopy (FIG. 15).

In summary, the present invention is highly industrially valuable by effectively overcoming various disadvantages of the prior art.

The above-described embodiments merely illustrate the principles and efficacy of the invention and are not intended to limit the invention. Modifications or variations of the embodiments described above will occur to those skilled in the art without departing from the spirit or scope of the invention. Accordingly, it is intended that the appended claims cover all such equivalent modifications and variations made by those of ordinary skill in the art without departing from the true spirit and scope of this invention.

## Claims

1. A lithographic method **characterized by** comprising the steps of:
1) providing first light and second light to a lithographic material, wherein the first light and the second light partially overlap, the lithographic material contains molecules for generating effector molecules controllable by a molecular switch, the first light is used for enabling the molecules for generating effector molecules controllable by the molecular switch to be in a turned-off state, and the second light is used for enabling the molecules for generating effector molecules controllable by the molecular switch to be in a turned-on state; the molecules for generating effector molecules controllable by the molecular switch in an area where the first light and the second light overlap are in the turned-off state; and at least part of the molecules for generating effector molecules controllable by the molecular switch in the turned-on state generate effector molecules, thereby changing physical and/or chemical properties of the lithographic material in the area where the molecular switch is turned on; and
2) removing either the lithographic material that has changed in physical or chemical properties or the lithographic material that has not changed.

2. The lithographic method according to claim 1, **characterized in that** one of the first light and the second light is single hollow light, and the other one of the first light and the second light at least partially covers a non-illuminated area surrounded by an area illuminated by one of the first light and the second light.

3. The lithographic method according to claim 1, **characterized in that** one of the first light and the second light is multiple hollow light, and the other one of the first light and the second light at least partially covers a non-illuminated area surrounded by an area illuminated by the first light.

4. The lithographic method according to claim 3, **characterized in that** at least one of the first light and the second light is array light.

5. The lithographic method according to any one of claims 3 to 4, **characterized in that** at least one of the first light and the second light is multiple light beams.

6. The lithographic method according to any one of claims 2 to 5, **characterized in that** an area illuminated by the other one of the first light and the second light does not exceed an outer edge of an area illuminated by one of the first light and the second light.

7. The lithographic method according to any one of claims 1 to 6, **characterized in that** the effector molecules generated by the molecules for generating effector molecules controllable by the molecular switch are selected from the group consisting of molecules for removing protecting groups from the lithographic material.

8. The lithographic method according to claim 7, **characterized in that** the molecules for removing protecting groups from the lithographic material are selected from acidic molecules, basic molecules, and singlet oxygen.

9. The lithographic method according to any one of claims 1 to 6, **characterized in that** the effector molecules generated by the molecules for generating effector molecules controllable by the molecular switch are selected from the group consisting of molecules for activating polymerization control of the lithographic material.

10. The lithographic method according to claim 9, **characterized in that** the molecules for activating polymerization control of the lithographic material are selected from polymerization initiation molecules.

11. The lithographic method according to any one of claims 7 to 10, **characterized in that** the molecules for generating effector molecules controllable by the molecular switch comprise a molecular switch group and an effector molecule generating group in a molecular structure thereof.

12. The lithographic method according to claim 11, **characterized in that** the molecular switch group is linked to the effector molecule generating group through a chemical bond.

13. The lithographic method according to any one of claims 1 to 12, **characterized in that** in step 1), the manner in which the molecules for generating effector molecules controllable by the molecular switch in the turned-on state generate the effector molecules refers to changing illumination conditions to which the molecules for generating effector molecules controllable by the molecular switch in the turned-on state are subjected.

14. The lithographic method according to any one of claims 1 to 13, **characterized in that** a change in physical or chemical properties of the lithographic material refers to a change in solubility in a developing solution.

15. The lithographic method according to any one of claims 1 to 14, **characterized in that** a method of changing the physical or chemical properties of the lithographic material is selected from the group consisting of removing protecting groups from the lithographic material and polymerizing polymer monomers in the lithographic material.

16. A product produced by the lithographic method according to any one of claims 1 to 15.

17. A lithographic material, **characterized by** comprising molecules for generating effector molecules controllable by the molecular switch and a compound sensitive to the effector molecule.

18. The lithographic material according to claim 17, **characterized in that** the effector molecules are selected from the group consisting of molecules for removing protecting groups from the lithographic material and molecules for activating polymerization control of the lithographic material.

19. The lithographic material according to claim 18, **characterized in that** the molecules for removing protecting groups from the lithographic material are selected from acidic molecules, basic molecules, and singlet oxygen.

20. The lithographic material according to claim 18, **characterized in that** the molecules for activating polymerization control of the lithographic material are selected from polymerization initiation molecules.

21. The lithographic material according to any one of claims 17 to 20, **characterized in that** the compound sensitive to the effector molecules is selected from the group consisting of a polymer sensitive to the effector molecules, a polymerized monomer, and an oligomer sensitive to the effector molecules.

22. The lithographic material according to any one of claims 17 to 21, **characterized in that** the molecule for generating effector molecules controllable by the molecular switch comprises a molecular switch group and an effector molecule generating group.

23. The lithographic material according to claim 22, **characterized in that** in the molecule for generating effector molecules controllable by the molecular switch, the molecular switch group is linked to the effector molecule generating group through a chemical bond.

24. A compound, **characterized by** comprising a molecular switch group and an effector molecule generating group.

25. The compound according to claim 24, **characterized in that** the effector molecules are selected from the group consisting of molecules for removing protecting groups from the lithographic material and molecules for activating polymerization control of the lithographic material.

26. The compound according to claim 25, **characterized in that** the molecules for removing protecting groups from the lithographic material are selected from acidic molecules, basic molecules, and singlet oxygen.

27. The compound according to claim 25, **characterized in that** the molecules for activating polymerization control of the lithographic material are selected from polymerization initiation molecules.

28. The compound according to any one of claims 24 to 27, **characterized in that** in the molecules for generating effector molecules controllable by the molecular switch, the molecular switch group is linked to the effector molecule generating group through a chemical bond.
